# EUROPÄISCHE PATENTSCHRIFT

(11) **EP 2 759 534 B1**
(45) Veröffentlichungstag und Bekanntmachung des Hinweises auf die Patenterteilung: **11.03.2015**
(21) Anmeldenummer: 13152696.4
(22) Anmeldetag: 25.01.2013
(51) Int. Cl.: C07D 211/58

(54) **4-N-(disubstituierte)-Aminopiperininderivateals Additive für Polyamidformmassen und deren Verwendung**
4-N-(disubstituted)-Aminopiperidine derivatives as additives for polyamide moulding material and their use
Derivés de 4-N-(disubstitués)-aminopipéridine comme additifs pour masses de formage en polyamide et leur utilisation

(43) Veröffentlichungstag der Anmeldung: 30.07.2014
(73) Patentinhaber: Uhde Inventa-Fischer GmbH, 13509 Berlin (DE)
(72) Erfinder: Katzer, Dr. Johannes, 10405 Berlin (DE)
(74) Vertreter: Pfenning, Meinig & Partner GbR

(56) Entgegenhaltungen:
- EP-A1- 0 047 967
- EP-A1- 0 488 502
- EP-A1- 0 839 854
- WO-A1-97/05189
- DE-A1- 2 719 132
- DE-A1- 19 804 980
- DE-A1- 19 812 135
- US-A- 4 145 512
- US-A1- 2002 169 181

## Beschreibung

Die Erfindung betrifft ein Additiv mit mindestens zwei kondensationsfähigen Funktionalitäten in Kombination mit einem Tetraalkyl-piperidinyl-Rest sowie einer tertiären Aminfunktionalität. Durch die Kombination dieser Funktionalitäten kann ein universelles Additiv bereitgestellt werden, das zum einen eine schmalere Molmassenverteilung ermöglicht und gleichzeitig die Performance für gesponnene Polymere verbessert. Erfindungsgemäß werden ebenso entsprechende additivierte Polyamidformmassen sowie eine Additiv-Lösung bereitgestellt. Verwendung finden die erfindungsgemäßen Additive insbesondere bei der Herstellung von Polyamid für textile Anwendungen.

Für den Einsatz von Polyamid für textile Anwendung existiert eine Reihe von Anforderungen, die diese Systeme erfüllen müssen:
• Mit den stetig gestiegenen Durchsätzen in Spinnereianlagen (High-Speed-Spinning) sind auch die Anforderungen an das Polymer gestiegen. Es wird eine gute Verspinnbarkeit bei geringen Fadenbruchraten erwartet.
• Damit die hergestellten Fäden ihre mechanischen und optischen Eigenschaften möglichst lange behalten, wird eine Resistenz gegenüber Luftsauerstoff und natürlichen Witterungsbedingungen gewünscht.
• Damit das Polymer beim Wiederaufschmelzen und während des Spinnereiprozesses seine physikalischen Eigenschaften möglichst wenig ändert, wird eine hohe Schmelzestabilität gefordert (geringe Monomer-Rückbildung und geringe Nachkondensation).
• Die Fäden müssen gut anfärbbar sein.
• Additive zum Erreichen der gewünschten Performance müssen mit einem wirtschaftlichen Kosten-Nutzen-Verhältnis erhältlich und applizierbar sein.

Für eine gute Verspinnbarkeit, besonders bei hohen Abzugsgeschwindigkeiten, hat sich der Einsatz von multifunktionellen Stabilisatoren bewährt, welche einen verschmälernden Einfluss auf die Molmassenverteilung haben. Vor allem werden multifunktionale Carbonsäuren eingesetzt, welche gleichzeitig den Gehalt an primären Amin-Gruppen im Kondensationsgleichgewicht gering halten und damit günstig für eine geringe Monomer-Rückbildung beim Wiederaufschmelzen sind. Beispielhaft für den Einsatz von multifunktionalen Säuren als Additive für die Polyamid-Herstellung seien EP 0 818 491 A1 und EP 0 759 953 A1 genannt. Der verschmälernde Einfluss auf die Molmassenverteilung ist z.B. dem Fachartikel von Z.-L. Tang et al. erschienen in "Die Angewandte Makromolekulare Chemie" (250, 1-14, Nr. 4321, Jahr 1997) zu entnehmen. Die Tatsache, dass sich eine schmalere Molmassenverteilung günstig auf die Spinn-Performance auswirkt, wurde z.B. in DE 10 2008 026 075 oder im Fachartikel von S. S. Raje et al. erschienen in "Man-Made Textiles in India", p. 173-178, 1996, beschrieben.

Für die antioxidativen Eigenschaften hat sich die Verwendung des 2,2,6,6-Tetraalkylpiperidinyl-Restes bewährt (sog. HALS-Verbindung), wie er z.B. über das 4-Amino-2,2,6,6-Tetramethylpiperidin (Kurzname Triacetondiamin, Abkürzung TAD, siehe z.B. DE 19 854 421 A1) oder das unter dem Handelsnamen Nylostab-SEED vertriebene Additiv applizierbar ist (siehe z.B. DE 10 2008 026 075 A1 oder F. P. La Mantia et al. in "Polymers For Advanced Technologies", 16, 2005, 357-361). Grundsätzlich ist es sowohl möglich, die funktionelle Gruppe des 2,2,6,6-Tetraalkylpiperidinyl-Restes in Form einer zusätzlichen inerten Verbindung einzubringen oder aber an das Polymer zu binden. Ersteres hat den Nachteil, dass das Additiv mit der Zeit ausgewaschen werden kann, letzteres ist bislang nur mit quasi-monofunktionellen, gleichzeitig als Kettenlängenregler fungierenden, aber die Molmassenverteilung nicht modifizierenden Verbindungen möglich, wie z.B. mit dem genannten 4-Amino-2,2,6,6-Tetramethylpiperidin.

Um eine hohe Schmelzstabilität zu gewährleisten, ist es wichtig, die Zahl an reaktiven Kettenenden zu minimieren. Dies verringert die Geschwindigkeit einer Nachkondensation und einer Monomer-Rückbildung z.B. beim Wiederaufschmelzen von Polyamid 6. Der mengenmäßige Einsatz von Kettenlängenreglern wird durch die verfahrenstechnischen Möglichkeiten und die Berücksichtigung ökonomischer Faktoren begrenzt.

Da Amino-Endgruppen für eine gute Anfärbung wichtig sind, werden, wie z.B. in EP 0 818 491 A1 beschrieben, tertiäre Amine ins Polymer eingebracht. Diese können keine Amid-Bindung eingehen und bleiben deshalb für die Anfärbung erhalten, unabhängig vom Reaktionsgeschehen. Auf diese Weise kann der Gehalt an kondensationsfähigen und für eine Monomer-Rückbildung relevanten primären Amin-Gruppen beliebig reduziert werden, gleichzeitig bleibt aber eine gute Anfärbbarkeit gewährleistet.

Die WO 97/05189 betrifft licht- und hitzestabilisierte Polyamide, in die Tetralkylpiperidin-4-Aminderivate eingebunden werden. Die DE 198 12 135 A1 betrifft ein Verfahren zur Herstellung von licht- und hitzestabilisierten Polyamiden, wobei die Stabilisierung durch Einbau von Piperidin-Derivaten ermöglicht wird.

Da die Rohstoffkosten den Großteil des Marktpreises von Polyamid ausmachen, sind die Zusatzmöglichkeiten von Additiven, die deutlich teurer sind als das entsprechende Monomer (Caprolactam), begrenzt. Die Mehrkosten, die durch die Additivierung entstehen, müssen durch eine höhere Performance und einen höherwertigen Werkstoff kompensiert werden.

Ausgehend hiervon war es Aufgabe der vorliegenden Erfindung, ein universelles Additiv bereit zu stellen, das sowohl die Funktion eines Kettenlängenreglers als auch die eines thermischen Stabilisators aufweist, gleichzeitig günstig hinsichtlich einer Anfärbung wirkt und die Molmassenverteilung verschmälern kann.

Diese Aufgabe wird durch das Additiv gemäß Anspruch 1, die Additiv-Lösung mit den Merkmalen des Anspruchs 5 sowie der Polyamidformmasse mit den Merkmalen des Anspruchs 7 gelöst. In Anspruch 12 werden erfindungsgemäße Verwendungen angegeben. Die weiteren abhängigen Ansprüche zeigen vorteilhafte Weiterbildungen auf.

Erfindungsgemäß wird ein Additiv mit mindestens zwei kondensationsfähigen Funktionalitäten der allgemeinen Formel I bereitgestellt:

Ein bevorzugtes erfindungsgemäßes Additiv weist die allgemeine Formel II auf: 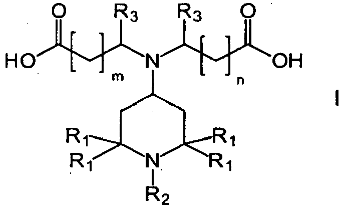 Hierbei bedeuten
R₁ und R₂ jeweils unabhängig voneinander H oder C₁-C₁₂-Alkyl
R₃ unabhängig voneinander H oder ein aliphatisches oder ungesättigtes oder aromatisches Kohlenstoffgerüst, linear oder verzweigt, mit 1 bis 12 Kohlenstoffatomen und
m und n jeweils unabhängig voneinander 0 bis 10.

Das erfindungsgemäße Additiv sorgt für eine schmalere Molmassenverteilung verglichen mit einer teilweisen oder vollständigen Stabilisierung mit monofunktionellen Kettenlängenreglern. Die HALS-Verbindung sowie das tertiäre Amin werden nicht wie bisher üblich ans Kettenende gebunden, sondern in die Kette eingebaut und reduzieren damit, gleiche verfahrenstechnische Bedingungen vorausgesetzt, nicht mehr das Potential die Polydispersität zu senken. Ein Performance-Gewinn im Bereich des High-Speed-Spinnings ist deshalb zu erwarten.

Das erfindungsgemäße Additiv ermöglicht erstmals, die genannten Anforderungen an textiles Polyamid mit einer einzigen Verbindung bedienen zu können. Damit können Dosierstrecken und Lagertanks auf ein Minimum reduziert werden und gleichzeitig verringert sich die Zahl an Lieferanten, von denen die Produktion abhängig wird. Die Strukturen sind auf Basis bestehender Massen chemikalien herstellbar und es sind keine Mehrkosten zu bestehenden, vergleichbaren Mehrkomponenten-Rezepturen zu erwarten. Die genannten Strukturen ermöglichen erstmals den Einbau der HALS-Verbindung bzw. des tertiären Amins in die Polymerkette. Bisher ist über quasi-monofunktionelle Verbindungen nur die Bindung ans Kettenende möglich, was die Möglichkeiten der Modifizierung der Molmassenverteilung begrenzt. Durch den Gewinn an Potential, die Molmassenverteilung schmaler zu gestalten, sind Performance-Erhöhungen im Bereich des High-Speed-Spinnings möglich, da sich die Schmelzefestigkeit verringert, die Festigkeit im erstarrten Zustand aber erhöht.

Vorzugsweise weist mindestens einer der Reste R₃ eine Carboxy-Funktionalität auf. Insbesondere ist mindestens einer der Reste R₃ CH2COOH.

Eine besonders bevorzugte Ausführungsform des erfindungsgemäßen Additivs weist die Formel II auf:

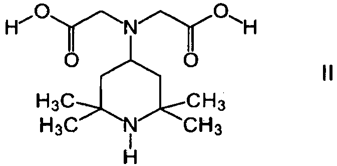

Erfindungsgemäß wird ebenso eine Additiv-Lösung bereitgestellt, die das zuvor beschriebene Additiv zusammen mit Caprolactam enthält. Weitere Additive können in dieser Lösung enthalten sein.

Vorzugsweise kann die Additiv-Lösung zusätzlich Terephthalsäure und/oder Adipinsäure enthalten.

Erfindungsgemäß wird ebenso eine Polyamidformmasse bereitgestellt, die ein Additiv aufweist, wobei das Additiv über mindestens zwei kondensationsfähige Carboxy-Funktionalitäten in der Polymerkette kovalent gebunden ist.

Eine bevorzugte Ausführunggsform sieht vor, dass Einheiten der allgemeinen Formel III in die Polymerkette eingebaut sind: 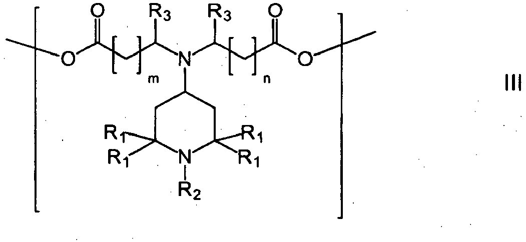 mit
R₁ und R₂ jeweils unabhängig voneinander H oder C₁- C₁₂-Alkyl,
R₃ unabhängig voneinander H oder ein aliphatisches oder ungesättigtes oder aromatisches Kohlenstoffgerüst, linear oder verzweigt, mit 1 bis 12 Kohlenstoffatomen und
m und n jeweils unabhängig voneinander 0 bis 10.

Besonders bevorzugt sind Einheiten der Formel IV in die Polymerkette eingebaut:

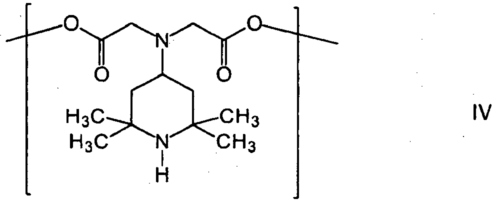

Dabei ist es bevorzugt, dass die Polyamidformmasse eine Polydispersität im Bereich im Bereich von 1,3 bis 2,5, insbesondere 1,5 bis 2,0 aufweist.

Verwendung findet das erfindungsgemäße Additiv, wie er zuvor beschrieben wurde, bei der Herstellung von Polykondensaten, insbesondere für textile Anwendungen.

Anhand der nachfolgenden Figuren und Beispiele soll der erfindungsgemäße Gegenstand näher erläutert werden, ohne diesen auf die hier gezeigten spezifischen Ausführungsformen einschränken zu wollen.
Fig. 1 zeigt anhand eines Diagramms die Rückbildung von Caprolactam in Abhängigkeit von der Zeit.
Fig. 2 zeigt anhand eines Diagramms die relative Viskosität in Abhängigkeit von der Zeit.

### Vergleichsbeispiel 1 (VB1)

Caprolactam wird bei einem konstanten Wassergehalt von 300 mmol/kg (0.54 w-%) bei 245°C bis zum Erreichen des Polykondensationsgleichgewichtes polymerisiert. Anschließend wird das Polymer granuliert und mit heißem Wasser überschüssiges Caprolactam bis auf 0.1 w-% (8.84 mmol/kg) extrahiert. Über eine Trocknung im Stickstoffstrom wird anschließend der Wassergehalt bis auf 0.05 w-% (27.8 mmol/kg) gesenkt.

### Vergleichsbeispiel 2 (VB2)

Caprolactam und 41.7 mmol/kg Essigsäure werden bei einem konstanten Wassergehalt von 125 mmol/kg (0.225 w-%) bei 245°C bis zum Erreichen des Polykondensationsgleichgewichtes polymerisiert. Die weitere Verarbeitung erfolgt analog zu Vergleichsbeispiel 1.

### Vergleichsbeispiel 3 (VB3)

Caprolactam, 33.4 mmol/kg Terephthalsäure und 17.5 mmol/kg Triacetondiamin werden bei einem konstanten Wassergehalt von 125 mmol/kg (0.225 w-%) bei 245°C bis zum Erreichen des Polykondensationsgleichgewichtes polymerisiert. Die weitere Verarbeitung erfolgt analog zu Vergleichsbeispiel 1.

### Beispiel 1 (erfindungsgemäß, B1)

Caprolactam, 19.03 mmol/kg Terephthalsäure und 17.5 mmol/kg Verbindung III werden bei einem konstanten Wassergehalt von 125 mmol/kg (0.225 w-%) bei 245°C bis zum Erreichen des Polykondensationsgleichgewichtes polymerisiert. Die weitere Verarbeitung erfolgt analog zu Vergleichsbeispiel 1.

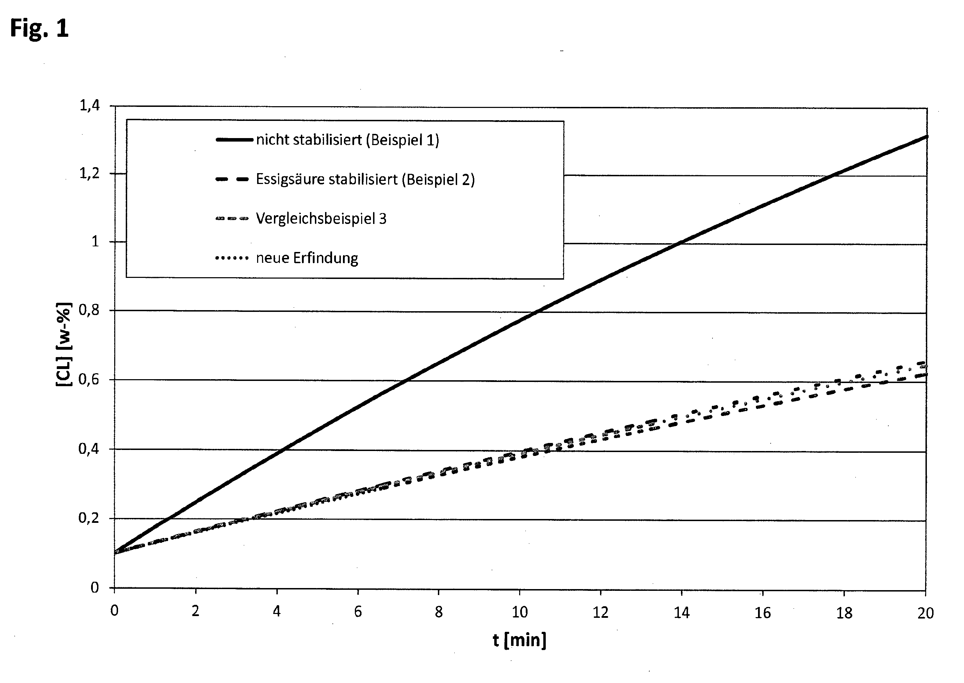

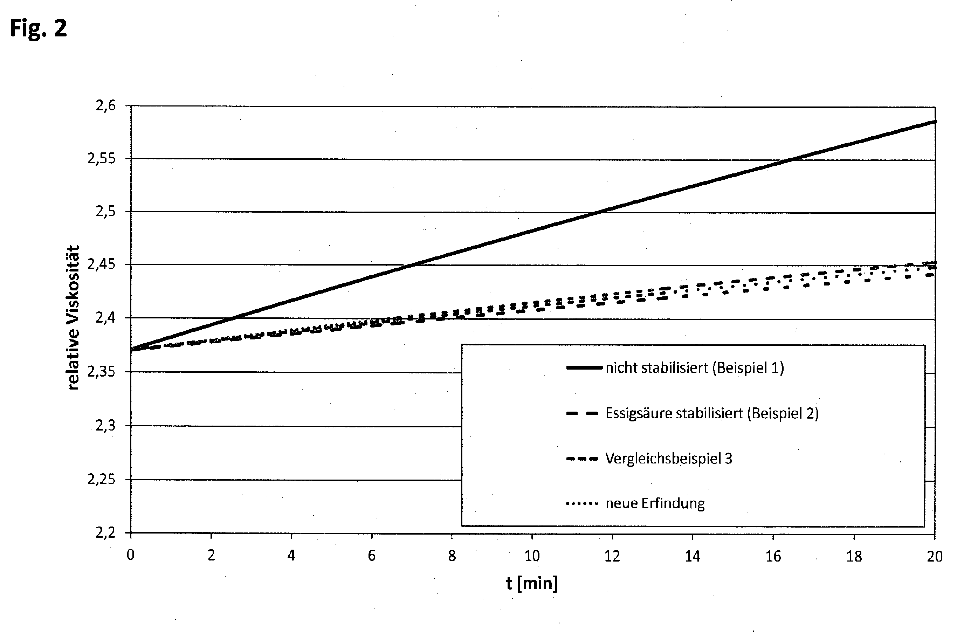

Tabelle 1 stellt die Eigenschaften der synthetisierten Polymere der Vergleichsbeispiele 1 bis 3 und des erfindungsgemäßen Beispiels 1 gegenüber. Alle besitzen die gleiche relative Viskosität, welche als Maß für die Verarbeitbarkeit im geschmolzenen Zustand herangezogen werden kann. Werden die 4 Polymere unter identischen Bedingungen bei 260°C aufgeschmolzen und die Rückbildung von Caprolactam sowie die Veränderung der relativen Viskosität mit der Zeit verfolgt, ergeben sich die in Fig. 1 und Fig. 2 dargestellten Ergebnisse. Daraus geht hervor, dass für eine geringe Rückbildung von Caprolactam, welches als Weichmacher agiert und damit die physikalischen Eigenschaften verändert sowie beim Spinnen zu unerwünschter Rauchbildung führen kann, genauso wie für eine geringe Nachkondensation die Verwendung von Stabilisatoren zwingend erforderlich ist. Die Art des Stabilisators ist dabei praktisch unerheblich. Für textile Anwendungen höchster Anforderung werden allerdings zusätzlich eine gute antioxidative Wirkung, eine gute Anfärbbarkeit und eine hohe Festigkeit des Polymers gefordert. Die Verwendung von Verbindung II in Kombination mit Terephthalsäure (B1) ermöglicht im Gegensatz zur Kombination aus Triacetondiamin und Terephthalsäure (VB3) unter identischen Reaktionsbedingungen und bei gleicher Beständigkeit gegenüber Witterungsbedingungen (gleiche Konzentration an HALS-Resten) sowohl eine Verringerung der Polydispersität (erhöhte Festigkeit im erstarrten Zustand) und eine höhere Konzentration an Amin-Endgruppen, was wiederum für eine Anfärbung günstig ist.

**Tabelle 1:**

| Probe | rel. Visk.^{*)} | Amin-Endgruppen [mmol/kg] | Carboxyl-Endgruppen [mmol/kg] | HALS-Rest [mmol/kg] | Polydispersität |
|---|---|---|---|---|---|
| VB1 | 2.37 | 71.41 | 71.41 | - | 2.0 |
| VB2 | 2.37 | 29.74 | 71.44 | - | 2.0 |
| VB3 | 2.37 | 45.12 | 76.92 | 17.5 | 1.71 |
| B1 | 2.37 | 57.28 | 95.34 | 17.5 | 1.65 |

| | | | | | |
|---|---|---|---|---|---|
| ^{*)} gemessen als 1 w-%ige Lösung in konzentrierter Schwefelsäure | | | | | |

## Patentansprüche

1. Additiv mit mindestens zwei kondensationsfähigen Funktionalitäten mit der allgemeinen Formel I: 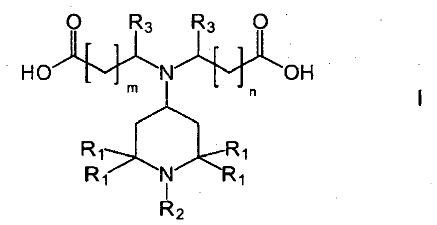 mit
R₁ und R₂ jeweils unabhängig voneinander H ₒder _{C1-}C₁₂-Alkyl R₃ unabhängig voneinander H oder ein aliphatisches oder ungesättigtes oder aromatisches Kohlenstoffgerüst, linear oder verzweigt, mit 1 bis 12 Kohlenstoffatomen und
m und n jeweils unabhängig voneinander 0 bis 10.

2. Additiv nach Anspruch 1,
**dadurch gekennzeichnet, dass** mindestens einer der Reste R₃ eine Carboxy-, eine Amin- oder eine Alkohol-Funktionalität aufweist.

3. Additiv nach Anspruch 1,
**dadurch gekennzeichnet, dass** mindestens einer der Reste R₃=CH₂COOH.

4. Additiv nach einem der vorhergehenden Ansprüche mit der Formel II:

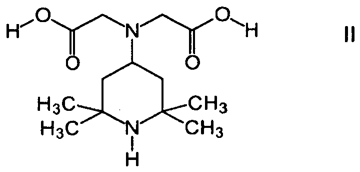

5. Additiv-Lösung enthaltend Caprolactam sowie ein Additiv nach einem der vorhergehenden Ansprüche.

6. Additiv-Lösung nach Anspruch 5,
**dadurch gekennzeichnet, dass** die Lösung zusätzlich bifunktionelle Carbonsäuren, insbesondere Terephthalsäure oder Adipinsäure, sowie Mischungen hiervon enthält.

7. Polyamidformmasse enthaltend ein Additiv nach einem der Ansprüche 1 bis 4, wobei das Additiv über mindestens zwei Carboxy-Funktionalitäten in der Polymerkette kovalent gebunden ist.

8. Polyamidformmasse nach Anspruch 7,
**dadurch gekennzeichnet, dass** Einheiten der allgemeinen Formel III in die Polymerkette eingebaut sind: 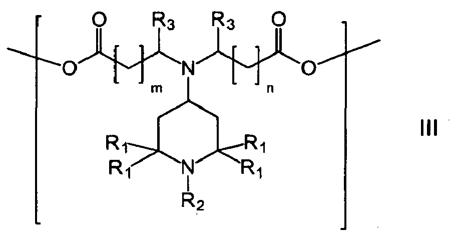 mit
R₁ und R₂ jeweils unabhängig voneinander H oder C₁- C₁₂-Alkyl,
R₃ unabhängig voneinander H oder ein aliphatisches oder ungesättigtes oder aromatisches Kohlenstoffgerüst, linear oder verzweigt, mit 1 bis 12 Kohlenstoffatomen und
m und n jeweils unabhängig voneinander 0 bis 10.

9. Polyamidformmasse nach einem der Ansprüche 7 oder 8,
**dadurch gekennzeichnet, dass** Einheiten der allgemeinen Formel IV in die Polymerkette eingebaut sind:

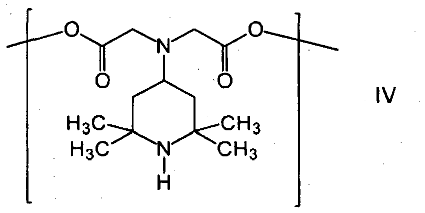

10. Polyamidformmasse nach einem der Ansprüche 7 bis 9,
**dadurch gekennzeichnet, dass** die Polyamidformmasse eine Polydispersität im Bereich von 1,3 bis 2,5, insbesondere 1,5 bis 2,0 aufweist.

11. Verwendung des Additivs nach einem der Ansprüche 1 bis 4 für die Herstellung von Polykondensaten.

12. Verwendung nach Anspruch 11 für die Herstellung von Polyamidformmassen, insbesondere für textile Anwendungen.

## Claims

1. Additive having at least two functional groups which are capable of condensation, having the general formula I: 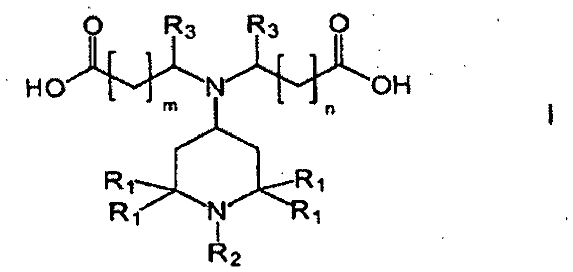 where
R₁ and R₂ are each, independently of one another, H or C₁-C₁₂ alkyl,
R₃ are, independently of each other, H or an aliphatic or unsaturated or aromatic carbon chain, linear or branched, having 1 to 12 carbon atoms and
m and n are, independently of each other, 0 to 10.

2. Additive according to claim 1,
**characterised in that** at least one of the radicals R₃ has a carboxyl, an amine or an alcohol functional group.

3. Additive according to claim 1,
**characterised in that** at least one of the radicals R₃ = CH₂COOH.

4. Additive according to one of the preceding claims, having the formula II:

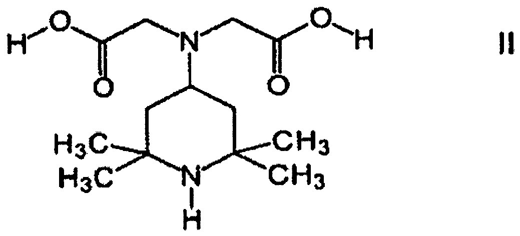

5. Additive solution containing caprolactam as well as an additive according to one of the preceding claims.

6. Additive solution according to claim 5, **characterised in that** the solution additionally contains bifunctional carboxylic acids, in particular terephthalic acid or adipic acid, as well as mixtures thereof.

7. Polyamide compound containing an additive according to one of claims 1 to 4, wherein the additive is bonded covalently in the polymer chain via at least two carboxyl functional groups.

8. Polyamide compound according to claim 7, **characterised in that** the units of the general formula III are incorporated into the polymer chain: 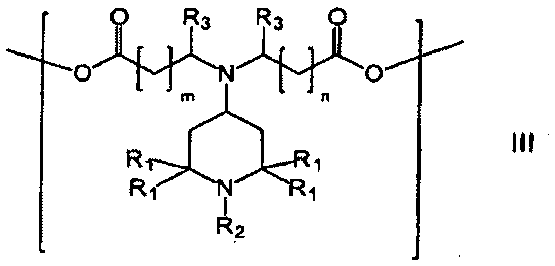 where
R₁ and R₂ are each, independently of one another, H or C₁-C₁₂ alkyl,
R₃ are, independently of each other, H or an aliphatic or unsaturated or aromatic carbon chain, linear or branched, having 1 to 12 carbon atoms and
m and n are, independently of each other, 0 to 10.

9. Polyamide compound according to one of claims 7 or 8, **characterised in that** units of the general formula IV are incorporated into the polymer chain:

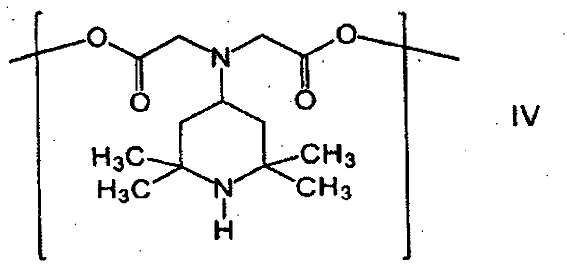

10. Polyamide compound according to one of claims 7 to 9, **characterised in that** the polyamide compound has a polydispersity in the range from 1.3 to 2.5, in particular 1.5 to 2.0.

11. Use of the additive according to one of claims 1 to 4 for the production of polycondensates.

12. Use according to claim 11 for the production of polyamide compounds, in particular for textile applications.

## Revendications

1. Additif comprenant au moins deux fonctionnalités condensables de formule générale 1 : 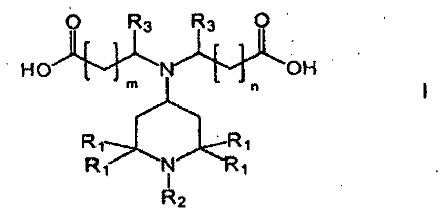 dans laquelle
R₁ et R₂ représentent chacun indépendamment l'un de l'autre H ou alkyle en C₁-C₁₂,
les R₃ représentent indépendamment les uns des autres H ou un squelette carboné aliphatique ou insaturé ou aromatique, linéaire ou ramifié, de 1 à 12 atomes de carbone, et m et n représentent chacun indépendamment l'un de l'autre 0 à 10.

2. Additif selon la revendication 1, **caractérisé en ce qu'**au moins un des radicaux R₃ présente une fonctionnalité carboxy, amino ou alcool.

3. Additif selon la revendication 1, **caractérisé en ce qu'**au moins un des radicaux R₃ = CH₂COO.

4. Additif selon l'une quelconque des revendications précédentes, de formule II :

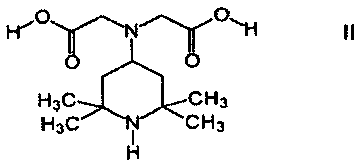

5. Solution d'additif contenant du caprolactame et un additif selon l'une quelconque des revendications précédentes.

6. Solution d'additif selon la revendication 5, **caractérisée en ce que** la solution contient en outre des acides carboxyliques bifonctionnels, notamment de l'acide téréphtalique ou de l'acide adipique, ainsi que leurs mélanges.

7. Matériau de moulage à base de polyamide contenant un additif selon l'une quelconque des revendications 1 à 4, l'additif étant relié de manière covalente dans la chaîne polymère par au moins deux fonctionnalités carboxy.

8. Matériau de moulage à base de polyamide selon la revendication 7, **caractérisé en ce que** des unités de formule générale III sont incorporées dans la chaîne polymère : 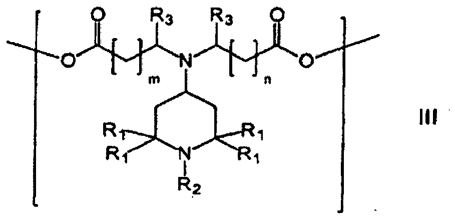 dans laquelle
R₁ et R₂ représentent chacun indépendamment l'un de l'autre H ou alkyle en C₁-Ci₂,
les R₃ représentent indépendamment les uns des autres H ou un squelette carboné aliphatique ou insaturé ou aromatique, linéaire ou ramifié, de 1 à 12 atomes de carbone, et m et n représentent chacun indépendamment l'un de l'autre 0 à 10.

9. Matériau de moulage à base de polyamide selon la revendication 7 ou 8, **caractérisé en ce que** des unités de formule générale IV sont incorporées dans la chaîne polymère :

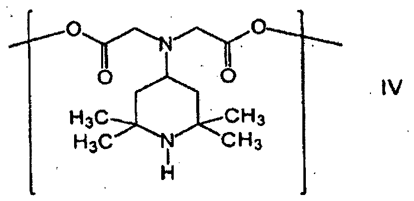

10. Matériau de moulage à base de polyamide selon l'une quelconque des revendications 7 à 9, **caractérisé en ce que** le matériau de moulage à base de polyamide présente une polydispersité dans la plage allant de 1,3 à 2,5, notamment de 1,5 à 2,0.

11. Utilisation de l'additif selon l'une quelconque des revendications 1 à 4 pour la fabrication de polycondensats.

12. Utilisation selon la revendication 11 pour la fabrication de matériaux de moulage à base de polyamide, notamment pour des applications textiles.
